# EUROPEAN PATENT SPECIFICATION

(11) **EP 1 944 378 B1**
(45) Date of publication and mention of the grant of the patent: **11.08.2010**
(21) Application number: 07150099.5
(22) Date of filing: 19.12.2007
(51) Int. Cl.: G01N 33/53, G01N 33/543, C12Q 1/68

(54) **Diagnostic and screening method**
Diagnose- und Screening-Verfahren
Diagnostic et procédé de criblage

(30) Priority: 27.12.2006 FI 20065857
(43) Date of publication of application: 16.07.2008
(73) Proprietor: ANI BIOTECH OY, 01720 Vantaa (FI); Pulmonary Cell Research Laboratory (PCRL), 4031 Basel (CH)
(72) Inventor: Gencay, Mikael, 4031 Basel (CH); Rüdiger, Jochen, 4031 Basel (CH); Roth-Chiarello, Michael, 4031 Basel (CH)
(74) Representative: Lax, Monica Ingeborg

(56) References cited:
- JP-A- 2 031 691
- US-A1- 2004 248 210
- TULLY, D.B. AND CIDLOWSKI, J.A.: "Characterization of Human Glucocorticoid Receptor Complexes Formed with DNA Fragments Containing or Lacking Glucocorticoid Response Elements" BIOCHEMISTRY, vol. 28, 1989, pages 1968-1975, XP002485157
- TULLY, D.B. AND CIDLOWSKI, J.A.: "Affinity of Interactions between Human Glucocorticoid Receptors and DNA: At Physiologic Ionic Strength, Stable Binding Occurs Only with DNAs Containing Partially Symmetric Glucocorticoid Response Elements" BIOCHEMISTRY, vol. 29, 1990, pages 6662-6670, XP002485158

## Description

### Background of the invention

Glucocorticoids or corticosteroids, such as cortisol, dexamethasone, hydrocortisone, methylprednisolone, prednisone, prednisolone and like, are in frequent use to treat diseases of inflammatory or immunological origin due to their function similar to the naturally occurring cortisol hormone. The cortisol and glucocorticoids act via binding to a glucocorticoid receptor (GR). Virtually all cells express glucocorticoid receptors and thus respond to glucocorticoid treatment. Therefore, besides the intended target cells or target tissues, all other cells are affected when systemic glucocorticoid treatment has to be administered to a patient. In addition, glucocorticoid treatment often has to be continued for weeks and even for years, which may result in serious and sometimes life threatening adverse effects. Clinicians adjust the glucocorticoid dosage to patients' body weight but cannot eliminate the severe side effects. Adverse effects include osteoporosis, muscle weakness, cataract, hypertension, and diabetes.

The glucocorticoid receptor belongs to a family of intracellular steroid hormone receptors, which control gene transcription by acting as transcription factors. In general, transcription factors bind to specific DNA sequences, thereby regulating the transcription of a certain gene. To achieve this, the transcription factors have to become activated first, whereafter their conformation changes and they translocate from the cytosol to DNA in the nucleus. When present in the cytosol, glucocorticoid receptors, stabilized by heat shock proteins, are in inactive state. When activated through binding of, for instance, cortisol or glucocorticoids, the glucocorticoid receptors form dimers with each other and are translocated into the nucleus, where they bind to specific palindromic DNA sequences.

By the measurement of the glucocorticoid receptor translocation and the ability of the glucocorticoid receptors to bind to specific corresponding DNA-sequences, it is possible to measure the potency of corticosteroids and the response of patients to glucocorticoid treatment in blood cells or other cells.

At present several methods are available for the measurement of glucocorticoid action. The potency of glucocorticoids may be determined by the measurement of binding affinity to the glucocorticoid receptor as disclosed by Smith CL and Kreutner W, Arzneimittelforschung 1998; 48(9): 956-60. This method can easily be automated. However, the interpretation of the data is of limited significance, since also GR antagonists bind to the glucocorticoid receptors, although they do not lead to similar pharmacological effects. Moreover, the binding affinity does not represent the biological activity.

*De novo* synthesis of the glucocorticoid receptor can be assessed by the measurement of the glucocorticoid receptor mRNA by various methods, for instance, by the polymerase chain reaction (PCR) as described in Korn SH, et al., Biochem Pharmacol 1998; 56(12): 1561-9. However, this method neither determines the amount nor the function of the glucocorticoid receptor.

The total amount of the glucocorticoid receptor protein can be measured by a Western blot analysis, which is the current standard procedure for measurement of the total amount of the glucocorticoid receptor. The specificity of the glucocorticoid receptor has to be determined with the help of specific antibodies. The Western blot analysis is specific, but it is time consuming and does not give information about the state of activation [Adcock IM, et al., Am J Respir Crit Care Med 1996; 154(3):771-82; Andersson O, et al., J Allergy Clin Immunol 1999; 103(4):595-600].

Enzyme-linked immunosorbent assays (ELISA) are less specific but quantitative. They are fast and can easily be automated. However, the standard sandwich ELISA methods cannot distinguish between the active and inactive form of glucocorticoid receptor [Radojcic M, et al., J Steroid Biochem 1985; 23(1):1-8; Flach H, et al., J Steroid Biochem Mol Biol 1992; 42(5):467-74].

Electrophoretic mobility shift assay (EMSA) is used to identify proteins that interact with DNA, such as GRs. By the EMSA technique it is possible detect active glucocorticoid receptors, which bind specifically to certain DNA sequences. Inactive glucocorticoid receptors cannot, however, be measured nor can quantitative results be obtained [Eickelberg O, et al., J Biol Chem 1999; 274(2):1005-10].

Vottero A, et al., J Clin Endocrinol Metab 2002; 87(6):2658-67, determine the GR activation by transfection of colors into living cells. However, this method is only suitable for *in vitro* systems, not for the determination of glucocorticoid receptor activation in patients.

Jiang X., et al., J Biol Chem 2004; 279(37):38480-5, describe a protein DNA array for the determination of glucocorticoid mediated activation of transcription factors. This method contains several complicated steps and it is not quantitative.

Consequently, none of the prior art methods provides a specific, quantitative measurement of a glucocorticoid receptor and is able to distinguish between the active and inactive forms of the glucocorticoid receptor.

Thus, new and efficient methods are still needed.

### Brief description of the invention

The present invention is based on the unexpected finding that the inactive form of a glucocorticoid receptor binds to any double stranded DNA sequence which is exploited in a method, which, as described herein, utilizes also a combination of existing technologies.

The purpose of the present invention is to provide means and methods, which allow a highly specific, quantitative measurement of the total amount of a glucocorticoid receptor as well as the amount of the active form of a glucocorticoid receptor, and, if desired, the amount of the inactive form of a glucocorticoid receptor in a biological sample.

Another purpose of the present invention is to provide means and methods, which are able to distinguish between the active and inactive forms of a glucocorticoid receptor.

Yet another purpose of the present invention is to provide means and methods, which enable a large-scale assay for the measurement of glucocorticoid action in general.

Yet another purpose of the present invention is to provide means and methods, which can be used in screening and developing of new drugs, which may mediate their main action or side effects through activation of a glucocorticoid receptor.

Yet another purpose of the present invention is to provide means and methods, which may help to identify the susceptibility of patients to glucocorticoids, to optimize the glucocorticoid dosage and possibly predict the patients' response to glucocorticoid treatment.

The purposes of the invention are achieved by a diagnostic and screening method, which takes advantage of known EMSA and ELISA methods by combining the specificity of EMSA to measure the active GR and the capability and sensitivity of ELISA to quantitatively measure the total GR.

The present invention relates to a diagnostic and/or screening method for quantitatively measuring the total and active glucocorticoid receptor (GR) in a biological sample comprising the steps of
a) contacting said sample, untreated or as pretreated by an excess of non-specific double stranded DNA, with a solid phase onto which a labeled DNA sequence, which specifically binds a glucocorticoid receptor, has been attached, and
b) measuring the amount of the bound glucocorticoid receptor, wherein said amount of the bound glucocorticoid receptor measured from the untreated sample represents the total GR and said amount of the bound glucocorticoid receptor measured from the pretreated sample represents the active GR.

Specifically, the labeled DNA sequence, which specifically binds the glucocorticoid receptor, is a labeled DNA sequence, which contains the conserved triplets of a glucocorticoid response element (GRE) and comprises the following general sequence:
5'- TGT ACA RRR TGT TCT -3' (SEQ. ID NO: 1),
wherein R is any one of nucleotides cytosine (C), thymine (T), adenine (A) and guanine (G).

The present invention also relates to a method for screening of a new drug, which may mediate their main action or their side effects through activation of the glucocorticoid receptor, wherein the above described method is performed using a biological sample which has been pretreated with said drug.

The present invention also relates to a method for identifying the susceptibility of a patient to glucocorticoids, wherein the above described method is performed using a series of biological samples obtained from said patient.

The present invention also relates to a method for optimizing the glucocorticoid dosage of a patient, wherein the above described method is performed using a series of biological samples obtained from said patient.

The present invention also relates to a method for predicting the response to glucocorticoid treatment in a patient, wherein the above described method is performed using a series of biological samples obtained from said patient.

The present invention also relates to a kit useful for carrying out the above methods.

### Drawings

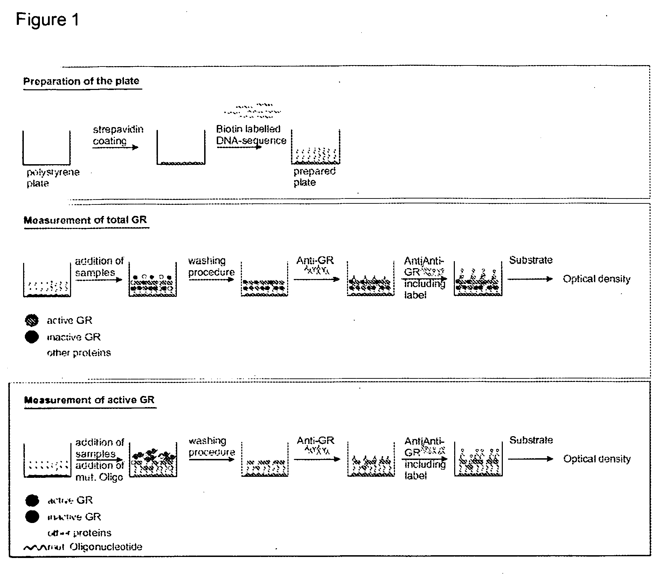

Figure 1 depicts one example of the method of the present invention: the assay is performed in two steps: (1) preparation of the plate and (2) detection of either the total and/or the active glucocorticoid receptor (GR). Anb-GR = anti-GR antibodies; mut. oligonucleotides = mutated oligonucleotides or dldc.

### Detailed description of the invention

Specific and sensitive measurement of the amount of active form of the glucocorticoid receptor provides a straightforward, precise and accurate method for assessing the level of glucocorticoid action. Assessment of glucocorticoid action, and thus also the active form of the glucocorticoid receptor, is the key to clinical and pharmacodynamic interpretation of the efficacy of glucocorticoid or cortisol treatment. The present invention is based on and utilizes the unexpected finding that the inactive form of a glucocorticoid receptor binds to any double stranded DNA sequence in contrast to the active glucocorticoid receptor, which only binds to a specific binding site on a double stranded DNA, namely the glucocorticoid response element (GRE). Thus, different DNA binding sequences can be used to distinguish between active and inactive form of the glucocorticoid receptor in the same assay. In addition, the present invention takes advantage of known EMSA and ELISA methods and combines the aspect of the protein-DNA interaction of the EMSA to measure the active glucocorticoid receptor (GR), and the capability of ELISA of a quantitative measurement.

Thus, the present invention is directed to a method for monitoring the efficacy of glucocorticoid therapy at a personal/individual level, to a method for the development of new glucocorticoidal and/or corticosteroidal molecules and/or drugs, and to a method for assessing the efficacy of glucocorticoidal and/or corticosteroidal molecules, the basis for these methods being that the inactive form of a glucocorticoid receptor binds to any double stranded DNA sequence.

The present invention relates to a diagnostic method for quantitatively measuring the total and active glucocorticoid receptor (GR) in a biological sample comprising the steps of
a) contacting said sample untreated or as pretreated with an excess of non-specific double stranded DNA with a solid phase onto which a labeled DNA sequence, which specifically binds a glucocorticoid receptor, has been attached, and
b) measuring the bound glucocorticoid receptor,
wherein said amount of the bound glucocorticoid receptor measured from the untreated sample represents the total GR and said amount of the bound glucocorticoid receptor measured from the pretreated sample represents the active GR.

In nature there is only one single DNA sequence that can specifically bind an active glucocorticoid receptor. This glucocorticoid response element (GRE) sequence occurs naturally in all gene promoters to which the glucocorticoid receptor can bind. No other GRE sequences are known to exist. The GRE sequence is embedded into the sequence of a gene promoter, which can vary from gene to gene, however this variation neither affects the binding affinity of the glucocorticoid receptor to the GRE nor its function. Glucocorticoid controlled genes include about 4500 genes or more, such as genes coding for enzymes of carbohydrate and amino acids metabolism, e.g. phosphoenol-pyruvate, tyrosine aminotransferase, tryptophan oxygenase; hormones, such as growth hormone, prolactin, angiotensinogen; hormone receptors, such as insulin receptor, glucocorticoid receptor, angiotensin; acute-phase proteins, such as interleukin-6; serum proteins, such as insulin-like growth factor binding protein 1, osteocalcin: milk proteins, such as casein. This specific DNA sequence consists of 5 triplets: the first 2 and the last 2 triplets are essential for the binding of the respective dimerized glucocorticoid receptors. The middle triplet consisting of any three nucleotides functions as a spacer between the four palindromic specific triplets.

The method of the present invention takes advantage of the specificity of the GRE and uses a labeled DNA sequence, which contains the conserved triplets of a glucocorticoid response element (GRE) and thus comprises the following general sequence:
5'- TGT ACA RRR TGT TCT -3' (SEQ. ID NO: 1),
wherein R is any one of nucleotides cytosine (C), thymine (T), adenine (A) and guanine (G).

The total length of the labeled DNA sequence can vary from 15 or more nucleotides, such as 15 to 60, preferably 15 to 30, most preferably 25 to 30 nucleotides.

One specific example of a GRE useful in the present invention is 5'-CTA GGC TGT ACA GGA TGT TCT GCC TAG A-3' (SEQ. ID NO: 2).

This sequence derives from the promoter sequence of the tyrosine aminotransferase.

The method of the present invention combines the specificity of the GRE to the novel finding that the inactive form of a glucocorticoid receptor binds to any double stranded DNA. The non-specific DNA sequence used in the present invention can be any double stranded DNA sequence of 15 or more nucleotides, such as 15 to 60, preferably 15 to 30, most preferably 15 to 20 nucleotides, which does not have the conserved triplets of the GRE and which does not adversely affect the method. Examples include commercially available artificial double stranded polynucleotides, such as dldC (e.g. Pharmacia Amersham, Clontech) or GRE sequences into which mutations have been introduced. Mutations can be such that they exchange a single or two neighboring nucleotides thereby reducing the glucocorticoid receptor binding specificity to zero or nearly zero. Alternatively mutations can be such that one or more natural nucleotides of the DNA sequences are replaced by less common or non-natural nucleotides, such as bromodesoxyuridine (brdU), hypoxanthine, 5-methylcytosine, inosin and like.

In a preferred embodiment of the invention a non-specific double stranded DNA sequence is 5'-CTA TAG GGA ACA TGT TGT TCT TGA GCC A-3' (SEQ. ID NO: 3).

To prepare the solid phase, the DNA sequence, which specifically binds the glucocorticoid receptor, is coupled to the solid phase by any suitable coupling system. Such systems are generally known in the art and contain a protein, which is coupled to the solid phase, and a corresponding means to detect this protein is used for the detection of the DNA sequence. Examples include avidin/streptavidin-biotin system. For instance, the solid phase is coated with streptavidin or avidin and the DNA sequence is labeled with biotin using standard procedures.

Suitable materials for the solid phase are synthetic materials, such as polystyrene, polyvinylchloride, polyamide and the other conventionally used synthetic polymers, as well as natural polymers, such as cellulose, derivatized natural polymers, such as cellulose acetate and nitrocellulose, paper and glass. The solid phase can be in a form of a microplate, a tube, a stick or a bead as conventionally used in the art. Also paper strips, plates or membranes may form a suitable solid phase useful in some embodiments of the invention. Preferred solid phases are microplates, tubes, sticks, paper, synthetic or nitrocellulose membrane strips. The most preferred materials are microplates of synthetic polymeric material, especially polystyrene, or nitrocellulose membranes.

The detection system used in the present invention can be any suitable automated immunoassay system, semi-automated immunoassay system or manual test system. Naturally, the detection system depends on the chosen label for the specific DNA sequence that binds the GR. In a preferred embodiment of the invention enzyme-linked immunosorbent assay (ELISA) is used.

The detection can be done using any reader system suitable for colorimetric including spectrometric, fluorometric, luminometric etc. assays. Specifically the reader is for 96-well microplates especially adapted for ELISA.

When the ELISA technique is used in the present invention, the binding of the glucocorticoid receptor to the labeled DNA sequence is detected using specific polyclonal or monoclonal antibodies, which can be enzyme-labeled. The label can be any suitable label, for example an acridinium ester, or produced by an enzymatic reaction with an enzyme selected from, for example, alkaline phosphatase, glucose oxidase, glucose-6-phosphate dehydrogenase, alpha-beta-galactosidase, horseradish peroxidase and xanthine oxidase.

When the enzyme used in the detection is alkaline phosphatase, its substrate is adamantyl 1,2-dioxetane aryl phosphate with or without enhancer or paranitrophenyl phosphate. In embodiments, where horseradish peroxidase is used as the enzyme, its substrate can be TMB (tetramethylbenzidine) or 2',3',6'-trifluorophenyl-3-methoxy-10-methylacridian-9-carboxylate.

The biological samples analyzed by the method of the invention contain cells, which respond to steroids and can derived from body fluids and tissues from a patient. The cells are isolated according to standard procedures and are optionally suitably pretreated. Examples of these cells include, fibroblasts, lymphocytes and epithelial cells. Where necessary, the cytosol and nuclear fractions can be separated with standard methods, such as those described in Example 2. Alternatively, the cell samples can derive from cultured cell lines, such as cultured human airway smooth muscle cells. The protein concentration of the sample is adjusted to contain from 5 to 25 µg, preferably from 10 to 15 µg protein, where necessary.

An embodiment of the method of the invention is exemplified in Fig. 1. Here, a 96-well microplate made of 12 strip polystyrene modules are used. The reaction wells were coated with streptavidin. The streptavidin-coated wells were further coated with a biotin-labeled DNA sequence of SEQ. ID NO: 2 containing the GRE of tyrosine amino transferase. The analysis was performed with a sample obtained from the same patient, whereby one portion of the sample was analyzed untreated and the other portion as pretreated with an excess of non-specific double stranded DNA. For the measurement of the total amount of the GR present in the sample to be analyzed, an untreated sample portion, a standard sandwich ELISA using polyclonal rabbit anti-GR antibodies as primary antibodies, horseradish peroxidase (HRP) conjugated antibodies as secondary antibodies, and TMB as the chromogenic molecule were used. For the measurement of the active form of the GR, the sample to be analyzed was first pretreated with an excess of non-specific double stranded DNA of SEQ ID NO: 3 and this mixture was used as the analysis sample and the same procedure as described above was performed. Only the active glucocorticoid receptor will now bind to the oligonucleotides fixed to the polystyrene surface and become measured. If desired, the amount of inactive GR can be calculated on the basis of the known amounts of total and active GR.

When nuclear and cytosolic cell fractions from primary human fibroblasts and lymphocytes were measured, the amount of the GR correlated to 95% with the results obtained for the same samples by a Western blot analysis, the current standard procedure for measurement of the total amount of the glucocorticoid receptor. However, the results obtained by the method of the invention are linear within a range from 2 ng to 60 pg/µl (Table 1) and thus reproducible between various experiments. In contrast, the Western blot analysis is not a linear test system, and although its detection limit is approx. 1 ng of a specific protein, it is semi-quantitative at best and indicates that the sample contains no protein at all, that the sample contains some protein or that the sample contains a lot of protein.

To distinguish between active and inactive forms of the glucocorticoid receptors, non-specific DNA oligo- or polynucleotides are added to the samples before the test. After the addition of non-specific DNA oligo- or polynucleotides the signals of the inactive glucocorticoid receptor are abolished, whereas signals of the active, nuclear glucocorticoid receptor are maintained. These results are comparable to the results obtained by a electrophoretic mobility shift assay (EMSA). By both methods, a significant increase in nuclear glucocorticoid receptor signal is obtained after dexamethasone treatment, which is known to activate the glucocorticoid receptor (Tables 2 and 3). Compared to the electrophoretic mobility shift assay, the method of the present invention is faster and much better for quantification. In conclusion, with the method of the present invention the specific binding activity of the active glucocorticoid receptor can be quantitatively detected and the total amount of the glucocorticoid receptor quantified in a fast process, which can be automated, if desired.

For reasons mentioned in the preceding paragraph, the method of the present invention can easily be adapted to large-scale DNA-binding assays for measurement of steroid action in general and also for the quantitative determination of the active forms of other DNA-binding receptors.

The method of the invention can be applied to control the glucocorticoid treatment and dosage at an individual patient level. This enables monitoring glucocorticoid therapy at a personal level within patients receiving such a theraphy. A high dose glucocorticoid treatment results in glucocorticoid receptor down regulation. Moreover, the glucocorticoid receptor activation in the patients may be impaired secondary to mutations of the glucocorticoid receptor or the functional status of the glucocorticoid receptor in the cytosolic and nuclear compartment, e.g. the interaction with inhibiting proteins such as the proteasome 26 [Wallace A.D. et al. JBC 267 (2001): 42714-21) or calreticulin [Bums K. et al. Nature 367 (1994): 476-80]. Thus, by measuring the amount of the glucocorticoid receptor after treating a patient sample with varying doses of a given drug, the glucocorticoid treatment and dosage can be adjusted to a desired efficient and safe level or in cases where no response is obtained, the useless glucocorticoid treatment can be withdrawn and other medication can be started.

The method of the invention can also be applied in the adjustment of the glucocorticoid dosage to maintain glucocorticoid action by measuring the amount of the activated glucocorticoid receptor used as a marker of glucocorticoid receptor tachyphylaxis. Additionally, the direct action on the glucocorticoid receptor pathway by inhaled or orally taken medication can be assessed, the time period needed for dose adjustment for a single patient could be reduced, and a number of adverse side effects could be avoided.

The test kit of the present invention comprises reagents needed to practice the method of the invention. The kit contains a solid support, which can be any solid support mentioned above, onto which the labeled specific DNA-sequence has been attached, non-specific DNA, and the reagents necessary for the detection. The kit may further contain to buffers and washing solution as well as positive and negative controls.

The invention is further illustrated by the following examples, however, without being limited to them.

### EXAMPLE 1. Preparation of the microplates

For the preparation of microplates a 96-well microplate consisting of 12 strips, each having 8 wells (Nunc A/S, Roskilde, Denmark) was used. The wells were coated with 5 µg/ml streptavidin (Sigma Chemicals, St. Louis, Missouri, USA) in 10 mmol/l phosphate buffered saline (NaCl 138 mmol/l; KCI 2.7 mmol/l), pH 7.4 (PBS) overnight at room temperature and washed 2x with 1xPBS+0.05% Tween-20. After washing, 150 µl of a saturation solution (1xPBS, 0.05% Tween-20, 0.5% BSA, and 6% D-sorbitol) was added to each well. The saturation solution was removed and the plates were air-dried.

The biotinylated double stranded glucocorticoid receptor-specfic oligonucleotides of SEQ.ID. NO. 1 were manufactured on request by MWG Biotech AG, Ebersberg, Germany. They were diluted in freshly prepared oligo-binding buffer (25 mM Tris-HCl, 125 mM NaCl, 5 mM EDTA, 5x Denhardt's solution, 0.1% Tween-20) to a concentration of 5 µg/ml and added into streptavidin-coated wells prepared above. After incubation for 1 hr at 37°C, the microplate was washed three times with an oligo wash buffer (25 mM Tris-HCl, 125 mM NaCl, 20 mM MgCl₂, and 0.1% Tween-20). The plates were used immediately or stored in the refrigerator at 4°C for up to 10 days.

### EXAMPLE 2. General principle of the measurement of the glucocorticoid receptor

First, for the calculation of the results a standard curve was created. A recombinant glucocorticoid protein (ABR Inc., CO, USA) was diluted to concentrations of 2, 1, 0.5, 0.25, 0.125 and 0.0625 ng/µl in 1xGR-protein diluent buffer (10 mM Hopes, 0.2 mM EDTA, 2.5 mM DTT, 40 mM KCI, 0.04% NP-40, 8% glycerol and 1x complete® protease inhibitor (Roche Diagnostics AG, Rotkreuz, Switzerland). 100 µl of each standard sample were applied to the reaction wells on the microplates prepared in Example 1. 100 µl of the dilution buffer were used as a blank and the analysis was performed in duplicate.

In order to measure the total amount of the glucocorticoid receptor, cytosolic and nuclear extracts of primary fibroblasts or lymphocytes from controls or patients were adjusted to contain 10-15 µg of total protein in 20 µl of 1xGR-protein diluent buffer. Samples from healthy persons without inflammation were used as controls in the Example. The samples were added to the reaction wells on the microplates prepared in Example 1 pre-filled with 80 µl GR-protein diluent buffer. The plates were incubated for 1 hr at room temperature under shaking at 300 rpm on a rotate shaker. After the incubation, the reaction wells were washed four times with 300 µl of a wash buffer (25mM Tris-HCl, 125mM NaCl, 20mM MgCl₂, 0.1% Tween-20, 5 µg/ml aprotinin (Sigma), and 1 mM PMSF (Sigma).

A rabbit polyclonal anti-GR antibody (Santa Cruz, Santa Cruz, CA) was diluted to a concentration of 0.5 µg/ml in an antibody diluent buffer (25 mM Tris-HCl, 125 mM NaCl, 2 mM Macl₂, 1% bovine serum albumine (BSA, Sigma), 0.1% Tween-20 and 1x complete® protease inhibitor), and 100 µl of the antibody solution was added to the reaction wells. The plates are incubated for 1 hr at room temperature and washed four times with 300 µl of the wash buffer using an automated plate washer (ThermoLabsystems, Helsinki, Finland).

A horseradish peroxidase (HPR) conjugated goat anti-rabbit antibody (DAKO, Glostrup, Denmark) was diluted 1:1000 in the antibody diluent buffer and 100 µl is added simultaneously to the reaction wells. The plates were incubated for 1 hr at room temperature and washed four times with 300 µl of the wash buffer. 50 µl of stabilized solution of TMB (tetramethylbenzidine) substrate (Roche Diagnostics AG, Rotkreuz, Switzerland) were added to the reaction wells and the plates were incubated for 30 min at room temperature in the dark.

The enzymatic reaction was stopped by adding 25 µl of 2 mol/l H₂SO₄ into the reaction wells and the absorbance of the color reaction was measured with an ELISA plate reader at 450 nm. The results of each test sample were quantified with the help of the standards using a software program (Excel, Microsoft, Redmond, WA). The detection limit of the test was set to 60 µg/µl based on the results obtained with the recombinant glucocorticoid receptor protein.

An example of a standard curve is presented in Table 1.

**Table 1. Standard curve**

| **Recombinant GR ng/µl** | **OD value at 450 nm** |
|---|---|
| 2 | 2.098 |
| 1 | 1.554 |
| 0.5 | 0.789 |
| 0.25 | 0.411 |
| 0.125 | 0.274 |
| 0.0625 | 0.193 |

In order to measure the active form of the glucocorticoid receptor, the samples prepared as in Example 2 were pretreated by adding 1 µg of mutated oligonucleotides of SEQ.ID. NO. 3 or dldC (Pharmacia Amersham) to each sample and mixing gently for 5 minutes. If non-specific DNA oligonucleotides containing BrdU instead of thymidine are used, UV light exposure could be used to stabilize the reaction. The 20 µl of samples were added to the reaction wells pre-filled with 80 µl protein diluent buffer and the subsequent procedure is as described above.

### EXAMPLE 3. Measurement of glucocorticoid treated cells with the method of the invention

Primary lung fibroblast cells (i.e. cells that were not virus transformed and were isolated from a tissue specimen directly and passaged once) of human origin were treated with the glucocorticoid dexamethasone (10⁻⁶ to 10⁻⁸ M) and cytosolic and/or nuclear extracts were prepared at timepoints 0 min, 30 min, 1 hr, 3 hr and 6 hr as described by Roth M, et al., (2000) European Respiratory Journal 16 [3 Suppl], 2855, and explained therein under "Methods".

The protein concentrations of the samples were determined with Bradford assay [Bradford, M. M. (1976) Anal. Biochem. 72; 248] and 15 µg of nuclear and cytosolic extracts each were analyzed for the measurement of total and active glucocorticoid receptor with the method described in Example 2. The standard curve was prepared as in Example 2. The results are set forth in Table 2.

**Table 2. Measurement of total and active form of the glucocorticoid receptor In primary fibroblast cells after glucocorticoid treatment**

| **Cytosolic extracts** | A_{total GR} | A_{active GR} | A_{dldC} |
|---|---|---|---|
| Control | 0.497 | 0.166 | 0.141 |
| 30 min. | 0.334 | 0.186 | 0.096 |
| 1 hour | 0.349 | 0.162 | 0.065 |
| 3 hours | 0.281 | 0.171 | 0.075 |
| 6 hours | 0.399 | 0.176 | 0.072 |
| | | | |

| **Nuclear extracts** | A_{total GR} | A_{ctive GR} | A_{dldC} |
|---|---|---|---|
| Control | 0.482 | 0.299 | 0.109 |
| 30 min. | 0.943 | 0.609 | 0.405 |
| 1 hour | 1.3 | 0.964 | 0.738 |
| 3 hours | 1.091 | 0.845 | 0.546 |
| 6 hours | 0.992 | 0.916 | 0.574 |

The amounts of total and active glucocorticoid receptor were quantified with the help of standards using a software program (Excel, Microsoft). The amount of the inactive glucocorticoid receptor can be determined, if desired, by subtracting the amount of active GR from the amount of total GR.

For comparison, the same nuclear extracts were analyzed with EMSA and Western blot methods. In the EMSA procedure, the sequence of SEQ. ID. NO. 2 was incubated with the samples and the samples were separated electrophoretically on a polyacrylamide gel using standard procedure. The signals were detected using phosphor imaging and analyzed using a digital image analyzing program (IMAGE J). The results for the nuclear extracts are presented in Table 3 as arbitrary units. It was not possible to obtain any substantial signals with cytosol extracts in EMSA.

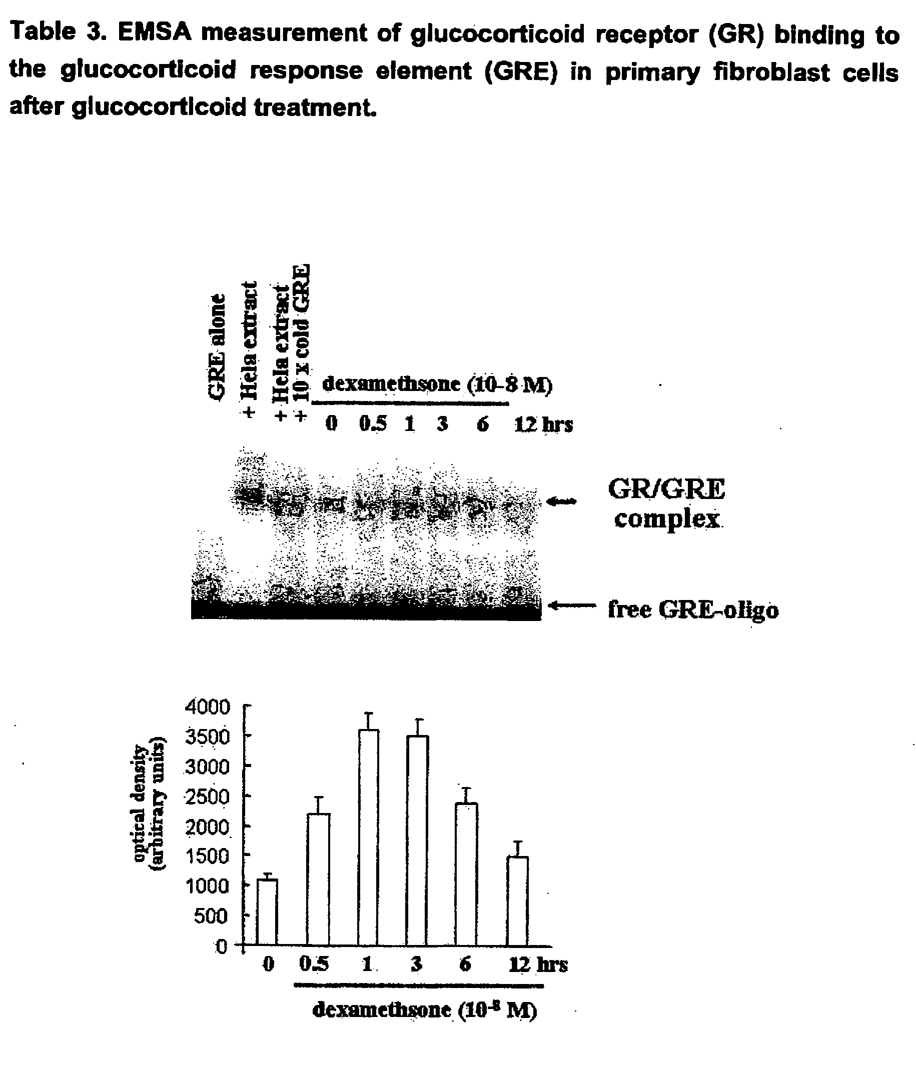

The Western blotting analysis of nuclear and cytosolic extracts of human fibroblasts after a single dose treatment with dexamethasone (10⁻⁸ M) was performed using anti-GR antibodies and anti-rabbit antibodies. The signals were detected using enhanced chemiluminescence (Pierce, Rockford, IL) and X-ray screens. The screens were scanned and signals were analyzed using a digital image analyzing program (IMAGE J). The results are presented in Table 4 as arbitrary units.

**Table 4. Western blot analysis of glucocorticoid receptor in primary fibroblast cells after glucocorticoid treatment**

| | **Nuclear extracts** | **Cytosolic extracts** |
|---|---|---|
| Control | 2200 | 2150 |
| 30 min. | 5000 | 5917 |
| 1 hour | 7917 | 6667 |
| 3 hours | 5167 | 2000 |
| 6 hours | 6583 | 2250 |

As can be seen from Tables 2, 3 and 4, the patterns of the glucocorticoid receptor activity obtained by the method of the invention are comparable to those obtained with Western blot and EMSA.

The comparison of Western-blot and EMSA data with that obtained by the invention can only be made on a semi-quantitative basis. Western-blot data and EMSA has to be converted into optical arbitrary units, which are calculated as a percentage of control conditions, set to 100% (e.g. untreated cells). Therefore a quantification based on a standard curve as done in the invention is impossible. When the same samples are used for Western-blot, EMSA and the present invention the relative changes of arbitrary units increase or decrease in a similar pattern. An advantage of the present invention is that according the method of the invention one can distinguish and quantify the active and the total glucocorticoid receptor.

### EXAMPLE 4. Comparison of a novel substance X with a known glucocorticoid

The method of the invention was used to compare a novel substance X, which is expected to differ in the potency and the length of the action when compared to known glucocorticoids exemplified here with budesonide Cultured primary human airway smooth muscle cells were treated in a time dependent manner (at 0 min, 30 min, 1 hr, 3 hr, 6 hr and 24 hr) using 10⁻⁸ M of the new substance X and budesonide. The drugs were dissolved in a lipophilic solvent and added to the cell culture medium.

After the treatment of the cells, nuclear and cytosolic extracts were separated and total GR was analyzed as described in Example 2. Cultured primary airway smooth muscle cells without treatment were used as a control. The results in pg/µl of GR are presented in Table 5.

**Table 5. Comparison of a novel substance X with budesonide**

| | Substance X | | Budesonide | |
|---|---|---|---|---|
| time | nuclear pg/µl of GR | cytosolic pg/µl of GR | nuclear pg/µl of GR | cytosolic pg/µl of GR |
| 0 | 94 | 242 | 94 | 242 |
| 30 min | 111 | 134 | 207 | 90 |
| 1 h | 211 | 134 | 339 | 51 |
| 3 h | 204 | 112 | 252 | 46 |
| 6 h | 209 | 63 | 107 | 37 |
| 24 h | 108 | 36 | 25 | 106 |

As evident from Table 5, the new substance X showed a slower but longer and stronger translocation of the glucocorticoid receptor into the nucleus than budesonide. This indicates a longer biological activity

### EXAMPLE 5. Comparison of a response to a β₂-agonist and a glucocorticoid

In the treatment of asthma β₂-agonists and glucocorticoids are used to control airway obstruction. In human cells β₂-agonists activate the glucocorticoid receptor *in vitro.* To test whether β₂-agonists activate the glucocorticoid receptor also *in vivo* and to test whether asthmatics and non-asthmatics differ in their response to the drugs applied, 3 volunteer asthmatics and 4 non-asthmatics were treated with a β₂-agonist formoterol, a glucocorticoid budesonide, a combination of budesonide and formoterol, and placebo (inactive substance or tracer element which is usually not identified by the manufacturer company), using 800 µg budesonide or 48 µg formoterol in a double blind, randomized trial. A blood sample was taken at 9 am and at 12 pm and lymphocytes were isolated by using a Ficoll gradient and subsequent cell counting. The nuclear and cytosolic extracts were separated and total GR was analyzed as described in Example 2. The results in pg/µl of GR are presented in Table 6.

**Table 6. Comparison of a response to a β₂-agonist and a glucocorticoid**

| Asthmatics | | | | |
|---|---|---|---|---|
| Timepoint | Placebo | Formoterol | Budesonide | Budesonide + formoterol |
| 9:00 a.m. | 630±28 | | | |
| 12:00 p.m. | 508±51 | 574±179 | 859±159 | 900±315 |
| | | | | |

| Non-asthmatics | | | | |
|---|---|---|---|---|
| Timepoint | Placebo | Formoterol | Budesonide | Budesonide + formoterol |
| 9:00 a.m. | 648±32 | | | |
| 12:00 p.m. | 366±69 | 664±230 | 545±203 | 737±94 |

Nuclear glucocorticoid receptor expression in 3 asthmatics was at 9:00 a.m. 630±28 pg/µl, naturally decreasing to 12:00 p.m. (508±51 pg/µl). The inhaled β₂-agonists formoterol increased the 12:00 p.m. nuclear GR abundance to 574±179 pg/µl, budesonide to 859±159 pg/µl and the combination of budesonide and formoterol to 900±315 µg/µl. In 4 non-asthmatics, nuclear GR abundance was not significantly different (baseline 648±32, placebo 366±69, formoterol 664±230 budesonide 545±203, combination of formoterol and budesonide 737±94 pg/µl). When the data was analyzed the following increase of the drugs' potency to activate the glucocorticoid receptor could be seen: Combination of formoterol and budesonide (830 pg/µl) > budesonide (724 pg/µl) >formoterol (613 pg/µl ) > placebo (460 pg/µl).

The potency of various substances causing glucocorticoid receptor accumulation in the nucleus of living cells *in vivo* was thereby measurable.

## Claims

1. A diagnostic method for quantitatively measuring the total and active glucocorticoid receptor (GR) in a biological sample comprising the steps of
a) contacting said sample untreated or as pretreated with an excess of non-specific double stranded DNA, which binds inactive glucocorticoid receptors, with a solid phase onto which a labeled DNA sequence specifically binding a glucocorticoid receptor has been attached, and
b) measuring the amount of bound glucocorticoid receptor,
wherein said amount of the bound glucocorticoid receptor measured from the untreated sample represents the total GR and said amount of the bound glucocorticoid receptor measured from the pretreated sample represents the active GR.

2. A method according to claim 1, wherein the labeled DNA sequence, which specifically binds the glucocorticoid receptor, is a labeled DNA sequence, which contains the conserved codons of a glucocorticoid response element (GRE) and comprises the following sequence:
5'- TGT ACA XXX TGT TCT -3' (SEQ. ID NO: 1),
wherein X is any one of nucleotides cytosine (C), thymine (T), adenine (A) and guanine (G).

3. A method according to claim 2, wherein the labeled DNA sequence, which specifically binds the glucocorticoid receptor, is a labeled DNA sequence of SEQ. ID NO: 2

4. A method according to claim 1, wherein the non-specific DNA sequence is a double stranded DNA sequence of 15 or more nucleotides, such as 15 to 60, preferably 15 to 30, most preferably 15 to 20 nucleotides, which does not have the conserved triplets of the GRE.

5. A method according to claim 4, wherein the non-specific DNA sequence is an artificial double stranded polynucleotide, such as dldC, or a GRE sequence into which mutations have been introduced.

6. A method according to claim 4, wherein the non-specific DNA sequence is SEQ. ID NO: 3.

7. A method for screening of a new drug, which may mediate the main action or side effects through activation of the glucocorticoid receptor, comprising the steps of
a) treating a biological sample with said drug,
b) contacting said sample, untreated or as pretreated by an excess of non-specific double stranded DNA, which binds inactive glucocorticoid receptors, with a solid phase onto which a labeled DNA sequence specifically binding a glucocorticoid receptor has been attached, and
b) measuring the bound glucocorticoid receptor,
wherein said amount of the bound glucocorticoid receptor measured from the untreated sample represents the total GR and said amount of the bound glucocorticoid receptor measured from the pretreated sample represents the active GR.

8. A method for identifying the susceptibility of a patient to glucocorticoids, comprising the steps of
a) contacting a series of biological samples obtained from a patient, said samples being untreated or pretreated by an excess of non-specific double stranded DNA, which binds inactive glucocorticoid receptors, with a solid phase onto which a labeled DNA sequence specifically binding a glucocorticoid receptor has been attached, and
b) measuring the bound glucocorticoid receptor,
wherein said amount of the bound glucocorticoid receptor measured from the untreated sample represents the total GR and said amount of the bound glucocorticoid receptor measured from the pretreated sample represents the active GR.

9. A method for optimizing the glucocorticoid dosage of a patient, comprising the steps of
a) contacting a series of biological samples obtained from a patient, said samples being untreated or pretreated by an excess of non-specific double stranded DNA, which binds inactive glucocorticoid receptors, with a solid phase onto which a labeled DNA sequence specifically binding a glucocorticoid receptor has been attached, and
b) measuring the bound glucocorticoid receptor,
wherein said amount of the bound glucocorticoid receptor measured from the untreated sample represents the total GR and said amount of the bound glucocorticoid receptor measured from the pretreated sample represents the active GR.

10. The test kit useful in the practice of a method of any one of claims 1 to 9, comprising a solid support, onto which a labeled specific DNA-sequence containing SEQ ID NO: 1 has been attached, double-stranded non-specific DNA having SEQ ID NO: 3, and the buffers and substrates necessary for the detection.

## Patentansprüche

1. Ein diagnostisches Verfahren zum quantitativen Messen des vollständigen und aktiven Glukokortikoid-Rezeptors (GR) in einer biologischen Probe, umfassend die Schritte von
a) Inkontaktbringen besagter Probe im unbehandelten Zustand bzw. in Form einer mit einem Überschuss einer nicht-spezifischen doppelsträngigen DNA, welche inaktive Glukokortikoid-Rezeptoren bindet, vorbehandelten Probe, mit einer Festphase, auf welcher eine gelabelte DNA-Sequenz, welche spezifisch einen Glukokortikoid-Rezeptor bindet, angebunden worden ist, und
b) Messen der Menge des gebundenen Glukokortikoid-Rezeptors,
wobei besagte Menge des gebundenen Glukokortikoid-Rezeptors, gemessen aus der unbehandelten Probe, den gesamten GR repräsentiert und besagte Menge des gebundenen Glukokortikoid-Rezeptors, gemessen aus der vorbehandelten Probe, den aktiven GR repräsentiert.

2. Das Verfahren gemäß Anspruch 1, wobei die gelabelte DNA-Sequenz, die spezifisch den Glukokortikoid-Rezeptor bindet, eine gelabelte DNA-Sequenz ist, welche die konservierten Codons eines Glukokortikoid-Antwort-Elementes (GRE) enthält und die folgende Sequenz umfasst:
5'- TGT ACAXXX TGT TCT -3' (SEQ ID NO: 1),
wobei X irgendeines der Nukleotide Cytosin (C), Thymine (T), Adenin (A) bzw. Guanin (G) ist.

3. Das Verfahren gemäß Anspruch 2, wobei die gelabelte DNA-Sequenz, welche spezifisch den Glukokortikoid-Rezeptor bindet, eine gelabelte DNA-Sequenz von SEQ ID NO: 2 ist.

4. Das Verfahren gemäß Anspruch 1, wobei die nicht-spezifische DNA-Sequenz eine doppelsträngige DNA-Sequenz ist von 15 oder mehr Nukleotiden, wie z.B. 15 bis 60, vorzugsweise 15 bis 30, am meisten bevorzugt 15 bis 20 Nukleotide, wobei die Sequenz nicht die konservierten Triplets des GREs aufweist.

5. Das Verfahren gemäß Anspruch 4, wobei die nicht-spezifische DNA-Sequenz ein künstliches doppelsträngiges Polynukleotid ist, wie z.B. dldC, oder eine GRE-Sequenz, in welche Mutationen eingebracht worden sind.

6. Das Verfahren gemäß Anspruch 4, wobei die nicht-spezifische DNA-Sequenz SEQ ID NO: 3 ist.

7. Ein Verfahren zum Screenen nach einer neuen Wirksubstanz, welche die hauptsächliche Wirkweise oder Nebenwirkungen durch Aktivierung des Glukokortikoid-Rezeptors vermitteln kann, umfassend die Schritte von
a) Behandeln einer biologischen Probe mit besagter Wirksubstanz,
b) Inkontaktbringen von besagter Probe im unbehandelten Zustand bzw. in Form einer mit einem Überschuss einer nicht-spezifischen doppelsträngigen DNA, welche inaktive Glukokortikoid-Rezeptoren bindet, vorbehandelten Probe, mit einer Festphase, auf welcher eine gelabelte DNA-Sequenz, welche spezifisch einen Glukokortikoid-Rezeptor bindet, angebunden worden ist und
c) Messen des gebundenen Glukokortikoid-Rezeptors,
wobei besagte Menge des gebundenen Glukokortikoid-Rezeptors, gemessen aus der unbehandelten Probe, den gesamten GR repräsentiert und besagte Menge des gebundenen Glukokortikoid-Rezeptors, gemessen aus der vorbehandelten Probe, den aktiven GR repräsentiert.

8. Ein Verfahren zum Identifizieren der Empfänglichkeit eines Patienten gegen Glukokortikoide, umfassend die Schritte von
a) Inkontaktbringen einer Serie von biologischen Proben, erhalten von dem Patienten, wobei die Proben unbehandelt sind bzw. vorbehandelt durch einen Überschuss einer nicht-spezifischen DNA, welche an inaktive Glukokortikoid-Rezeptoren bindet, sind, mit einer Festphase, auf welche eine gelabelte DNA-Sequenz, die spezifisch einen Glukokortikoid-Rezeptor bindet, angebunden worden ist, und
b) Messen des gebundenen Glukokortikoid-Rezeptors,
wobei besagte Menge des Glukokortikoid-Rezeptors, gemessen aus der unbehandelten Probe, den gesamten GR repräsentiert und besagte Menge des gebundenen Glukokortikoid-Rezeptors, gemessen aus der vorbehandelten Probe, den aktiven GR repräsentiert.

9. Ein Verfahren zum Optimieren der Glukokortikoid-Dosierung eines Patienten, umfassend die Schritte von
a) Inkontaktbringen einer Serie von biologischen Proben, erhalten von einem Patienten, wobei besagte Proben unbehandelt sind bzw. vorbehandelt mit einem Überschuss von nicht-spezifischer doppelsträngiger DNA, welche inaktive Glukokortikoid-Rezeptoren bindet, sind, mit einer Festphase, auf welche eine gelabelte DNA-Sequenz, welche spezifisch einen Glukokortikoid-Rezeptor bindet, angebunden worden ist, und
b) Messen des gebundenen Glukokortikoid-Rezeptors,
wobei besagte Menge des gebundenen Glukokortikoid-Rezeptors, gemessen aus der unbehandelten Probe, den gesamten GR repräsentiert und besagte Menge des gebundenen Glukokortikoid-Rezeptors, gemessen aus der vorbehandelten Probe, den aktiven GR repräsentiert.

10. Ein Test-Kit, der verwendbar ist beim Durchführen eines Verfahrens gemäß irgendeinem der Ansprüche 1 bis 9, umfassend einen festen Träger, auf welchem eine gelabelte spezifische DNA-Sequenz enthaltend SEQ ID NO: 1 angebundenen worden ist, eine doppelsträngige nicht-spezifische DNA, welche SEQ ID NO: 3 aufweist und die Puffer und Substrate, die zum Nachweis nötig sind.

## Revendications

1. Un procédé de diagnostic pour mesurer de manière quantitative le récepteur glucocorticoïde (GR) total et actif dans un échantillon biologique, comportant les étapes consistant à :
a) mettre en contact ledit échantillon non traité ou tel que traité préalablement avec un excès d'ADN bicaténaire non spécifique, qui lie les récepteurs glucocorticoïdes inactifs, avec une phase solide sur laquelle une séquence d'ADN marqué liant spécifiquement un récepteur glucocorticoïde a été fixée, et
b) mesurer la quantité de récepteur glucocorticoïde lié,
ladite quantité de récepteur glucocorticoïde lié qui est mesurée à partir de l'échantillon non traité représentant le GR total et ladite quantité de récepteur glucocorticoïde lié mesurée à partir de l'échantillon traité préalablement représentant le GR actif.

2. Procédé selon la revendication 1, dans lequel la séquence d'ADN marqué, qui lie spécifiquement le récepteur glucocorticoïde, est une séquence d'ADN marqué, qui contient les codons conservés d'un élément de réponse aux glucocorticoïdes (GRE) et comporte la séquence suivante :
5'- TGT ACA XXX TGT TCT -3'(SEQ. ID NO: 1),
dans laquelle X est l'un quelconque des nucléotides cytosine (C), thymine (T), adénine (A) et guanine (G).

3. Procédé selon la revendication 2, dans lequel la séquence d'ADN marqué, qui lie spécifiquement le récepteur glucocorticoïde, est une séquence d'ADN marqué de séquence SEQ. ID NO: 2

4. Procédé selon la revendication 1, dans lequel la séquence non spécifique d'ADN est une séquence d'ADN bicaténaire de 15 nucléotides ou plus, spécifiquement de 15 à 60, de préférence de 15 à 30, de préférence encore de 15 à 20 nucléotides, qui n'a pas les triplets conservés du GRE.

5. Procédé selon la revendication 4, dans lequel la séquence non spécifique d'ADN est un polynucléotide bicaténaire artificiel, telle que le dldC, ou une séquence de GRE sur laquelle des mutations ont été introduites.

6. Procédé selon la revendication 4, dans lequel la séquence non spécifique d'ADN est la séquence SEQ. ID NO: 3.

7. Procédé pour le criblage d'un nouveau médicament, qui peut agir sur l'action principale ou les effets secondaires par l'activation du récepteur glucocorticoïde, comportant les étapes consistant à :
a) traiter un échantillon biologique avec ledit médicament,
b) mettre en contact ledit échantillon, non traité ou tel que traité préalablement par un excès d'ADN bicaténaire non spécifique, qui lie les récepteurs glucocorticoïdes inactifs, avec une phase solide sur laquelle une séquence d'ADN marqué liant spécifiquement un récepteur glucocorticoïde a été attaché, et
c) mesurer le récepteur glucocorticoïde lié,
ladite quantité de récepteur glucocorticoïde lié mesurée à partir de l'échantillon non traité représentant le GR total et ladite quantité de récepteur glucocorticoïde lié mesurée à partir de l'échantillon traité préalablement représentant le GR actif.

8. Procédé pour identifier la susceptibilité d'un patient aux glucocorticoïdes, comportant les étapes consistant à :
a) mettre en contact une série d'échantillons biologiques obtenus à partir d'un patient, lesdits échantillons étant non traités ou traités préalablement par un excès d'ADN bicaténaire non spécifique, qui lie les récepteurs glucocorticoïdes inactifs, avec une phase solide sur laquelle une séquence d'ADN marqué liant spécifiquement un récepteur glucocorticoïde a été fixée, et
b) mesurer le récepteur glucocorticoïde lié,
ladite quantité de récepteur glucocorticoïde lié mesurée à partir de l'échantillon non traité représentant le GR total et ladite quantité de récepteur glucocorticoïde lié mesurée à partir de l'échantillon traité préalablement représentant le GR actif.

9. Procédé pour optimiser le dosage glucocorticoïde d'un patient, comportant les étapes consistant à :
a) mettre en contact une série d'échantillons biologiques obtenus à partir d'un patient, lesdits échantillons étant non traités ou traités préalablement par un excès d'ADN bicaténaire non spécifique, qui lie les récepteurs glucocorticoïdes inactifs, avec une phase solide sur laquelle une séquence d'ADN marqué liant spécifiquement un récepteur glucocorticoïde a été fixée, et
b) mesurer le récepteur glucocorticoïde lié,
ladite quantité de récepteur glucocorticoïde lié mesurée à partir de l'échantillon non traité représentant le GR total et ladite quantité de récepteur glucocorticoïde lié mesurée à partir de l'échantillon traité préalablement représentant le GR actif.

10. Kit d'essai utile dans la mise en oeuvre d'un procédé selon l'une quelconque des revendications 1 à 9, comprenant un support solide, sur lequel une séquence d'ADN spécifique marqué contenant la séquence SEQ. ID NO: 1 a été fixée, de l'ADN non spécifique bicaténaire ayant la séquence SEQ. ID NO: 3, et des tampons et substrats nécessaires pour la détection.


### SEQUENCE LISTING

<110> Orgenium Laboratories Oy
<120> Diagnostic and screening method
<130> 2050911FI
<160> 3
<170> PatentIn version 3.1
<210> 1
   <211> 15
   <212> DNA
   <213> Artificial
<220>
   <221> misc_feature
   <222> (7)..(7)
   <223> r is a, t, c, or g
<220>
   <221> misc_feature
   <222> (8)..(8)
   <223> r is a, t, c, or g
<220>
   <221> misc_feature
   <222> (9)..(9)
   <223> r is a, t, c, or g
<400> 1
   tgtacarrrt gttct 15
<210> 2
   <211> 27
   <212> DNA
   <213> Artificial
<400> 2
   ctaggctgta caggatgttc tgcctag 27
<210> 3
   <211> 28
   <212> DNA
   <213> Artificial
<400> 3
   ctatagggaa catgttgttc ttgagcca 28